# NEW EUROPEAN PATENT SPECIFICATION

(11) **EP 1 663 127 B2**
(45) Date of publication and mention of the opposition decision: **15.02.2017**
(45) Mention of the grant of the patent: 24.04.2013
(21) Application number: 04782775.3
(22) Date of filing: 31.08.2004
(51) Int. Cl.: A61Q 19/00, A61K 8/81, A61K 8/34, A61K 8/39, A61K 8/35, A61K 8/37, A61K 8/40, A61K 8/49, A61K 8/58, A61K 8/86

(54) **SKIN CARE COMPOSITION**
HAUTPFLEGEZUSAMMENSETZUNG
COMPOSITION POUR LE SOIN DE LA PEAU

(30) Priority: 10.09.2003 US 501804 P; 29.07.2004 US 592336 P
(43) Date of publication of application: 07.06.2006
(73) Proprietor: THE PROCTER & GAMBLE COMPANY, Cincinnati, OH 45202 (US)
(72) Inventor: SUGINAKA, Yasuko, (NMN), Ashiya, Hyogo 659-0092 (JP)
(74) Representative: Engisch, Gautier
(86) International application number: PCT/US2004/028353
(87) International publication number: WO 2005/025522

(56) References cited:
- EP-A- 0 336 900
- EP-A- 0 819 419
- EP-A2- 1 192 938
- WO-A-02/05770
- WO-A-94/08555
- WO-A-96/07395
- WO-A-02/058665
- WO-A-02/092046
- FR-A- 2 795 079
- US-A- 5 167 950

## Description

### Field of Invention

The present invention relates to a skin care composition comprising: (a) hydrophilic gelling agent; (b) a first nonionic surfactant system having a HLB value of 10 or more; (c) a lower alcohol; (d) an oily component; and (e) water. The composition of the present invention provides less greasy and/or less tacky feel to the skin while containing an increased level of oily components.

### Background of the Invention

Many personal care products currently available to consumers are directed primarily to improving and/or maintaining the health and/or physical appearance of the skin.

Addition of oily components is known to be advantageous for improving and/or maintaining the health and/or physical appearance of the skin. Such oily components are, for example, emollients, silicones, and UV protecting agents. Skin care compositions comprising emollients and silicones provide softness, smoothness, and supplement of intercellular lipid to the skin. Skin care compositions comprising UV protecting agents prevent excessive scaling and texture changes of the stratum corneum by exposure of ultraviolet light. However, it is also known that skin care compositions comprising such oily components provide greasy and/or tacky feel to the skin, especially when the composition comprises an increased level of such oily components.

Oily components are generally contained in skin care composition in the form of emulsions and/or dispersions. The use of surfactants is known for emulsifying and/or dispersing the oily components. When increased amount of oily components is contained in skin care composition, it is a common way to contain an increased amount of surfactants to emulsify and/or disperse the oily components. However, increased level of surfactants also provides greasy and/or tacky feel.

For consumers who prefer fresh feel, such greasy and/or tacky feel is not desirable.

WO02/092046 discloses skin care composition comprising from about 0.01-5% of a hydrophilic gelling agent selected from the group of a carboxylic acid/carboxylate copolymer, from about 0.1-2% of a surfactant system comprising two or more non-ionic surfactants selected from the group consisting of polyoxyalkylene hydrogenated castor oils, a mixture of water and lower alcohol selected from the group of monohydric alcohols having 1 to 6 carbons and 0.01-5% of an emollient oil.

JP2001-192328 discloses cosmetic emulsions comprising 0.22% hydrophilic gelling agents, 7% of oils, 0.4% of a first non-ionic surfactant having a HLB more than 10, 5% of ethanol and water. The compositions described in D2 comprises also 3% of a humectant (propylene glycol) and a second surfactant which is polyethylene glycol monostearate 60E.

JP2001335460 discloses cosmetic emulsions comprising 0.6-1.3% of hydrophilic gelling agents, 5-30% of oils, 0.5-6% of a first non-ionic surfactant having a HLB more than 10 (polyoxyethylene sorbitan monooleate 20E), 5% of ethanol and water. The compositions described in D3 comprises also 10% of butylene glycol and 5% of glycerol as humectants and a second surfactant which is sorbitan sesquioleate.

Based on the foregoing, there exists a need for skin care compositions which provide less greasy and/or less tacky feel to the skin while containing an increased level of oily components.

None of the existing art provides all of the advantages and benefits of the present invention.

### Summary of the Invention

The scope of the present invention is defined by the appended claims.

### Detailed Description of the Invention

While the specification concludes with claims particularly pointing out and distinctly claiming the invention, it is believed that the present invention will be better understood from the following description.

Herein, "comprising" means that other steps and other ingredients which do not affect the end result can be added. This term encompasses the terms "consisting of" and "consisting essentially of".

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Herein, "mixtures" is meant to include a simple combination of materials and any compounds that may result from their combination.

While the specification concludes with claims which particularly point out and distinctly claim the invention, it is believed the present invention will be better understood from the following description.

### COMPOSITION

The composition of the present invention comprises a hydrophilic gelling agent, a high HLB first surfactant system, a lower alcohol, an oily component, and water. The compositions of the present invention preferably further contain a second surfactant system.

The present compositions can provide less greasy and/or less tacky feel to the skin while containing an increased level of oily components.

The use of hydrophilic gelling agent can provide less greasy and/or tacky feel, compared to other polymers. The use of lower alcohol can also provide less greasy and/or tacky feel.

The use of high HLB first surfactant system can provide less greasy and/or tacky feel, compared to low HLB surfactants. Without being limited to the theory, it is believed that, compared to low HLB surfactants, high HLB surfactants provide larger particle size of oily components when emulsifying and/or dispersing oily components in aqueous phase because of less compatibility of high HLB surfactants with oily components. It is believed that larger particles of oily components provide less creamy sensory to the skin, yet provide less greasy and/or less tacky feel to the skin.

Furthermore, without being limited to the theory, it is believed that the use of hydrophilic gelling agent, high HLB first surfactant system, and a lower alcohol can make an increased level of oily components emulsified and/or dispersed with a small amount of the surfactant system.

In the composition of the present invention, the weight ratio of the oily component to the first surfactant system is in the range of preferably from 10:1 to 100:1, more preferably from 15:1 to 90:1, still more preferably from 30:1 to 90:1. When other surfactants such as the second surfactant system are contained in the composition, the weight ratio of the oily component to total surfactants (total of the first surfactant system and other surfactants such as the second surfactant system) is in the range of preferably from 10:1 to 100:1, more preferably from 15:1 to 90:1, still more preferably from 30:1 to 90:1.

In the compositions of the present invention, total surfactants is contained at a level by weight of the composition of preferably from 0.001% to 2.0%, more preferably from 0.01% to 1.0%, more preferably from 0.05% to 0.8%.

Preferably, the compositions of the present invention are substantially free of other surfactants than the first and second surfactant system. In the present invention, "substantially free of other surfactants" means that the composition contain 0.001% or less, preferably 0% of other surfactants than the first and second surfactant system.

The composition of the present invention is suitable for topical use on human body skin, particularly suitable for facial skin. Typically, applications would be on the order of about once per day over such extended periods, while application rates can be up to about three times per day or more.

### HYDROPHILIC GELLING AGENT

The compositions of the present invention comprise a hydrophilic gelling agent, at a level of preferably from 0.001% to 5%, more preferably from 0.04% to 2%, still more preferably from 0.04% to 1 % by weight of the composition.

Without being bound by theory, the hydrophilic gelling agent is believed to provide a shear thinning property to the present composition. What is meant by shear thinning property is that a yield point exists within a typical shear stress applicable by the hand on the skin, and that the viscosity of the composition beyond the yield point significantly decreases to the extent such decrease is noticeable by the consumer. In a preferred embodiment, the composition of the present composition, prior to application of shear, has a suitable viscosity, preferably from 100 mPa•s to 40,000 mPa•s, more preferably from 500 mPa•s to 38,000 mPa•s, still preferably from 700 mPa•s to 35,000 mPa•s. The viscosity herein can be suitably measured by Brookfield LV at 20rpm at 25°C using either spindle #4, 5, 6 or 7 depending on the viscosity and the characteristic of the composition. In a highly preferred embodiment, the viscosity beyond the yield point is similar to that of water. The shear thinning property provides a low viscous composition directly upon use, thereby providing the feel of applying water to the skin.

Additionally, the hydrophilic gelling agent is capable of dispersing and stabilizing the oily components in an aqueous environment, so that such components do not separate out.

The hydrophilic gelling agents suitable for use herein include carboxylic acid/carboxylate copolymers, acrylate homopolymers, acrylate copolymers, and mixtures thereof.

The carboxylic acid/carboxylate copolymers herein are hydrophobically-modified cross-linked copolymers of carboxylic acid and alkyl carboxylate, and have an amphiphilic property. It is believed that, because of the alkyl group contained in the copolymer, the carboxylic acid/carboxylate copolymers do not make the composition undesirably sticky.

Suitable carboxylic acid/carboxylate copolymers herein are acrylic acid/alkyl acrylate copolymers having the following formula: 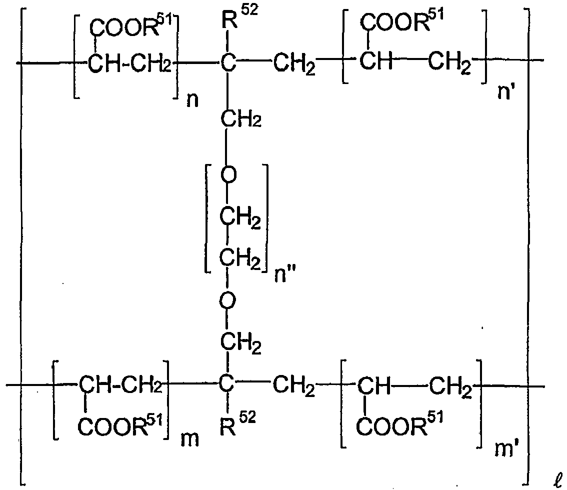 wherein R⁵¹, independently, is a hydrogen or an alkyl of 1 to 30 carbons wherein at least one of R⁵¹ is a hydrogen, R⁵² is as defined above, n, n', m and m' are integers in which n+n'+m+m' is from about 40 to about 100, n" is an integer of from 1 to about 30, and ℓ is defined so that the copolymer has a molecular weight of 500,000 to 3,000,000.

Commercially available carboxylic acid/carboxylate copolymers useful herein include: CTFA name Acrylates/C10-30 Alkyl Acrylate Crosspolymer having tradenames Carbopol Ultrez 21, Carbopol Ultrez 10, Pemulen TR-1, Pemulen TR-2, Carbopol 1342, Carbopol 1382, and Carbopol ETD 2020, all available from Noveon; and Ammonium Acryloyldimethyl taurate/beheneth-25 Methacrylate Crosspolymer having the tradename Aristoflex HMB, available from Clariant.

The acrylate homopolymers useful herein include sodium polyacrylate, polyethylacrylate, and polyacrylamide. Sodium Polyacrylate having the tradename Cosmedia SP, available from Cognis, is a preferred acrylate homopolymer.

The acrylate copolymers useful herein include PVP/VA copolymers, such as PVP/VA S-630 which is commercially available from ISP; and Ammonium Acryloyldimethyl taurate/VP Copolymer having the tradename Aristoflex AVC, available from Clariant.

Neutralizing agents may be included to neutralize the carboxylic acid/carboxylate polymer hydrophilic gelling agents, if needed. Nonlimiting examples of such neutralizing agents include sodium hydroxide, potassium hydroxide, ammonium hydroxide, monoethanolamine, diethanolamine, triethanolamine, diisopropanolamine, aminomethylpropanol, tromethamine, tetrahydroxypropyl ethylenediamine, and mixtures thereof.

### FIRST SURFACTANT SYSTEM

The composition of the present invention comprises a first surfactant system. The first surfactant system comprises one or more nonionic surfactants selected from the group consisting of polysorbates, polyoxyalkylene hydrogenated caster oils, polyglycerin alkyl esters having the C10-20 of alkylsubstitute, polyoxyethylene sterols, and polyoxyethylene hydrogenated sterols.

The hydrophilic-lipophilic balance (HLB) of the first surfactant system as a whole is 10 or more, preferably about 12 or more, more preferably from about 14 to about 20. Preferably, the first surfactant system consists essentially of nonionic surfactants having an HLB of 10 or more, more preferably 12 or more, still more preferably from about 14 to about 20.

Polyoxyalkylene hydrogenated castor oils useful herein include, for example, polyoxyethylene hydrogenated castor oils having 20-100 moles of ethylene oxides, such as polyoxyethylene (20) hydrogenated castor oil, polyethylene (40) hydrogenated castor oil, and polyoxyethylene (100) hydrogenated castor oil.

Polyglycerin alkyl esters having the C10-20 of alkylsubstitute useful herein include, for example, those having 6-10 moles of glycerin units, such as polyglyceryl-6 laurate, polyglyceryl-10 laurate, and polyglyceryl-10 stearate.

Polysorbates useful herein include, for example, those having 20-80 moles of ethylene oxides, such as polysorbate-20, polyborbate-40, polysorbate-60, and polysorbate-80.

Polyethylene sterols and polyethylene hydrogenated sterols useful herein include, for example, those having 10-30moles of ethylene oxides, such as polyethylene (10) phytosterol, polyethylene (30) phytosterol, and polyethylene (20) cholesterol.

Among the above nonionic surfactants, preferred are polysorbates, and more preferred are polysorbate-20, polysorbate-40, and mixtures thereof.

Preferably, the first surfactant system is liquid at 40°C, more preferably at 25°C.

The first surfactant system is contained in the composition of the present invention at a level of preferably from 0.001% to 1.0%, more preferably from 0.01% to 0.5%, still more preferably from 0.05% to 0.5% by weight of the composition.

### LOWER ALCOHOL

The composition of the present invention comprises a lower alcohol. Lower alcohols useful herein are monohydric alcohols having 1 to 6 carbons. The skin care compositions of the present invention comprise a lower alcohol selected from ethanol and isopropanol.

It is believed that the use of lower alcohols can provide less greasy and/or tacky feel. It is also believed that lower alcohols can help oily components dispersed with a small amount of the surfactant system.

Furthermore, lower alcohols can be used as preservatives. This is useful especially when oily components comprise polar oils such as UV protecting agents. Without being limited to the theory, it is believed that, when the compositions of the present invention contain such polar oils, some preservatives such as parabens tend to exist in the oil phase rather than water phases, thus it is difficult that the preservatives works efficiently in the water phase. It is also believed that the lower alcohol can exist in water phase and can be used as preservatives.

The lower alcohol can be contained in the composition at a level of preferably from : 3% to 20%, still more preferably from 5% to 15% by weight of the composition.

### OILY COMPONENT

The composition of the present invention comprises an oily component. The oily component useful herein include, for example, UV protecting agents, solvents of the UV protecting agents, emollients, silicone oils, and mixtures thereof. The oily component is contained in the composition at a level by weight of the composition of, preferably from 5% to 40%, more preferably from 10% to 25%.

### (i) UV protecting agent and solvents thereof

The UV protecting agents are those which generally prevent excessive scaling and texture changes of the stratum corneum by exposure of ultraviolet light. A wide variety of conventional UV protecting agent are suitable for use herein. Preferred are octyl methoxylcinnamate, octyl salicylate, octocrylene, avobenzone, homosalate, octyl triazone, and mixtures thereof. Other conventional UV protecting agents are also useful herein. Such agents include, for example, butylmethoxydibenzoyl-methane, 2-hydroxy-4-methoxybenzo-phenone, 2-phenylbenzimidazole-5-sulfonic acid, octyldimethyl-p-aminobenzoic acid, 2-ethylhexyl N,N-dimethyl-p-aminobenzoate, p-aminobenzoic acid, oxybenzone, 4-isopropyl dibenzoylmethane, 3-benzylidene camphor, 3-(4-methylbenzylidene) camphor.

Exact amounts will vary depending upon the sunscreen chosen and the desired Sun Protection Factor (SPF). SPF is a commonly used measure of photoprotection of a sunscreen against erythema. See Federal Register, Vol. 43, No. 166, pp. 38206-38269, August 25, 1978.

When the UV protecting agents are solid, it is preferred to dissolve them in a solvent in view of obtaining higher SPF. Such solvents are, generally hydrophobic. Preferred are isopropyl lauroyl sarcosinate, butyloctyl salicylate, diethyl hexyl 2,6-naphthalate, tricaprylin, and mixtures thereof. Solvent useful herein can be also used as the "Emollient" described below.

The UV protecting agents and solvents thereof are preferably used together with other oily components. When used together with other oily components, the UV protecting agents and solvents thereof are contained in the composition at a level of preferably from 0.1% t 30%, more preferably from 1% to 25%, still more preferably from 5% to 25% by weight of the composition.

### (ii) Emollient

The emollient oil useful herein are those having a melting point of not more than about 25°C, and provide emollient benefit to the skin. The emollient oils useful herein include esters and hydrocarbons. Emollient oils of lower viscosity, low molecular weight, or branched structure, are highly preferable. It has been surprisingly found that, by the use of such oils, the tacky and greasy feel to the skin can be alleviated.

Emollient oils useful herein are esters, particularly esters having branched alkyl and alkenyl groups. Such esters include, for example, cetyl 2-ethylhexanoate, tridecyl isononanoate, isostearyl isostearate, isocetyl isostearate, isopropyl isostearate, isodecyl isonoanoate, cetyl octanoate, isononyl isononanoate, diisopropyl myristate, isocetyl myristate, isotridecyl myristate, isopropyl myristate, myristyl myristate, isostearyl palmitate, isocetyl palmitate, isodecyl palmitate, isopropyl palmitate, isostearyl myristate, octyl palmitate, caprylic/capric acid triglyceride, glyceryl tri-2-ethylhexanoate, neopentyl glycol di(2-ethyl hexanoate), neopentyl glycol dicaprate, diisopropyl dimerate, glycerol trioctanate, glycerol triisopalmitate, isopropyl myristate, octyldodecyl lactate, and mixtures thereof. Triglycerides such as caprylic/capric triglyceride, PEG-6 caprylic/capric triglyceride, and PEG-8 caprylic/capric triglyceride may also be useful. Crude mixtures of such triglycerides by the CTFA name Meadowfoam seed oil are also useful. Commercially available oils include, for example, isononyl isononanoate with tradenames Salacos 99 available from Nisshin Oil Mills, or Lanol 99 available from Seppic; tridecyl isononanoate with tradename Crodamol TN available from Croda, and Hexalan available from Nisshin Seiyu, and Meadowfoam Seed Oil with tradename Cropure MDF available from Croda.

Emollient oils also useful herein are the various grades and types of hydrocarbons. Mineral oils are liquid mixtures of hydrocarbons that are obtained from petroleum. Specific examples of suitable hydrocarbons include paraffin oil including those called light paraffin or isoparaffin, mineral oil, squalane, dodecane, isododecane, hexadecane, isohexadecane, eicosene, isoeicosene, tridecane, tetradecane, hydrogenated polyisobutylene, docosane, and mixtures thereof. Commercially available hydrocarbons useful herein include isododecane, isohexadeance, and isoeicosene with tradenames PERMETHYL 99A, PERMETHYL 101A, and PERMETHYL 1082, available from Presperse (South Plainfield New Jersey, USA), mineral oil with tradename BENOL available from Witco, isoparaffin with tradename ISOPAR available from Exxon Chemical Co. (Houston Texas, USA.), tradename Isoparaffin 2028 available from Idemitsu, and tradename Amsco Mineral Spirits available from Ashland.

### (iii) Silicone oils

The silicone oils are preferably used together with other oily components. When used together with other oily components, the silicone oils are contained in the composition at a level of preferably from 0.01% to 10%, more preferably from 0.1 % to 5%, still more preferably from 0.5% to 3% by weight of the composition.

The silicone components useful herein include insoluble silicones suitable for use on the skin. By insoluble what is meant is that the silicone forms a separate, discontinuous phase from the carrier, such as in the form of an emulsion or a suspension of droplets of the silicone. The silicone components herein may be made by any suitable method known in the art, including emulsion polymerization. The silicone components may further be incorporated in the present composition in the form of an emulsion, wherein the emulsion is made my mechanical mixing, or in the stage of synthesis through emulsion polymerization, with or without the aid of a surfactant selected from anionic surfactants, nonionic surfactants, cationic surfactants, and mixtures thereof. Silicone components of high molecular weight may be made by emulsion polymerization.

Silicone components useful herein include polyalkyl polyaryl siloxanes, silicone resins, amino-substituted siloxanes, and mixtures thereof. The silicone component is preferably selected from the group consisting of polyalkyl polyaryl siloxanes, silicone resins, and mixtures thereof, and more preferably from one or more polyalkyl polyaryl siloxanes.

Polyalkyl polyaryl siloxanes useful herein include those with the following structure (I) 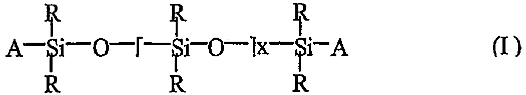 wherein R is alkyl or aryl, and x is an integer from about 1 to about 8,000. "A" represents groups which block the ends of the silicone chains. The alkyl or aryl groups substituted on the siloxane chain (R) or at the ends of the siloxane chains (A) can have any structure as long as the resulting silicone remains fluid at room temperature, is dispersible, is neither irritating, toxic nor otherwise harmful when applied to the skin, is compatible with the other components of the composition, is chemically stable under normal use and storage conditions, and is capable of being deposited on and conditions the skin. Suitable A groups include hydroxy, methyl, methoxy, ethoxy, propoxy, and aryloxy. The two R groups on the silicon atom may represent the same group or different groups. Preferably, the two R groups represent the same group. Suitable R groups include methyl, ethyl, propyl, phenyl, methylphenyl and phenylmethyl, trialkylsiloxanes such as trimethylsiloxane.

Among the above polyalkyl polyaryl siloxanes, highly preferred are those having the above formula (I), wherein x is an integer of from 1 to about 15, preferably from 1 to about 10, more preferably from 1 to about 5, and wherein at least one of R is phenyl. Such highly preferred polyalkyl polyaryl siloxanes include, for example, phenyl trimethicone, phenyl methicone, phenyl propyl trimethicone, and phenyl propyl trimethicone/diphenylmethicone. Commercially available highly preferred polyalkyl polyaryl siloxanes include, for example, phenyl trimethicone having a tradename Silicone oil KF56 available from Shinetsu Chem, and phenyl propyl trimethicone having a tradename DC2-2043 Cosmetic Grade Fluid available from Dow Corning. These silicone oils having a phenyl group are especially useful when the oily component comprising UV protecting agents. When containing UV protecting agents, the compositions generally provide draggy and/or dry feel when applied to skin. The use of the silicone oils having a phenyl group provides improved soft and smooth feel when applied to skin.

Another polyalkyl polyaryl siloxane that can be useful is a polyalkylsiloxane such as polydimethylsiloxane and polydiethylsiloxane. Polydimethylsiloxane is also known as dimethicone. These compounds are available, for example, from the General Electric Company in their Viscasil and SF 96 series, and from Dow Coming in their Dow Coming 200 series.

Another polyalkyl polyaryl siloxane that can be useful is a silicone gum. The term "silicone gum", as used herein, means a polyorganosiloxane material having a viscosity at 25°C of greater than or equal to 1,000,000 mm²s⁻¹ (1,000,000 centistokes). It is recognized that the silicone gums described herein can also have some overlap with the above-disclosed silicone compounds. This overlap is not intended as a limitation on any of these materials. The "silicone gums" will typically have a mass molecular weight in excess of about 200,000, generally between 200,000 and 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. High viscosity silicone compounds such as silicone gums are preferably used as together with silicone based carriers. Silicone based carriers include those having a viscosity of from about 6mPa•s to about 100mPa•s selected from cyclomethicones and dimethicones having lower repeating units.

### WATER

The compositions of the present invention comprise water. Deionized water is preferably used. Water from natural sources including mineral cations can also be used, depending on the desired characteristic of the product.

Generally, the composition of the present invention comprises from 10% to 99%, preferably from 50% to 95%, more preferably from 60% to 90% by weight of the composition, of water.

The pH of the present composition is preferably from 4 to 8, more preferably from 5 to 7. The suitable tacky skin treatment agents are particularly efficient in such pH range. Buffers and other pH adjusting agents can be included to achieve the desirable pH.

### SECOND SURFACTANT SYSTEM

The composition of the present invention preferably contains a second surfactant system. The second surfactant system comprises one or more nonionic surfactants selected from the group consisting of silicone copolyol esters, silicone copolyols, and mixtures thereof.

Silicone copolyol esters useful herein include, for example, those having from 2 to 10 dialkylsiloxane units preferably dimethylpolysiloxane units, having from 2 to 20 carbon atoms derived from a fatty acid, and having from 5 to 10 oxyalkylene groups. Such silicone copolyol esters include, for example, Dimethicone isostearate such as Dimethicone PEG-7 isostearate which is commercially available, for example, from Noveon with a tradename Ultrasil DW18, and Dimethicone olivate such as Dimethicone PEG-7 olivate which is commercially available, for example, from Noveon with a tradename Ultrasil DW-O.

Silicone copolyols, useful herein include, for example, those having from 2 to 10 dialkylsiloxane units preferably dimethylpolysiloxane units, and having from 2 to 10 oxyalkylene groups. Such silicone copolyol include, for example, Dimethicone copolyols such as PEG-10 Dimethicone which is commercially available, for example, from Shinetsu Chem. with a tradename KF-6017.

Preferably, the second surfactant system is liquid at 40°C, more preferably at 25°C.

The second surfactant system is contained in the composition of the present invention at a level of preferably from 0.001% to 1.0%, more preferably from 0.01% to 0.5%, still more preferably from 0.05% to 0.5% by weight of the composition.

### ADDITIONAL WATER SOLUBLE POLYMER

The compositions of the present invention may further contain an additional water soluble polymer, preferably at a level of from 0.01% to 5%, more preferably from 0.04% to 1%. The additional water soluble polymers herein are water soluble or water miscible polymers, and are compatible with the carboxylic acid/carboxylate copolymers. Without being bound by theory, it is believed the controlled amount of additional water soluble polymers in the composition provides improved moisturization and smoothness to the skin without giving an undesirable tacky or sticky feeling.

In one preferred embodiment, the additional water soluble polymer is selected so that the composition of the present composition has a suitable viscosity of preferably from 100 mPa•s to 6000 mPa•s, as described above.

Additional water soluble polymers useful herein include anionic polymers and nonionic polymers. Useful herein include, for example, vinyl polymers such as cross linked acrylic acid polymers with the CTFA name Carbomer; cellulose derivatives and modified cellulose polymers such as methyl cellulose, ethyl cellulose, hydroxyethyl cellulose, hydroxyethyl ethylcellulose, hydroxypropyl methyl cellulose; polyvinylpyrrolidone; polyvinyl alcohol; gums such as guar gum, hydroxypropyl guar gum, and xanthan gum; microbiological polymers such as dextran and succinoglucan; starch-based polymers such as carboxymethyl starch and methylhydroxypropyl starch. Polyalkylene glycols having a molecular weight of more than about 1000 are also useful herein. Additional water soluble polymers also useful herein include amphoteric polymers such as Polyquaternium 22, Polyquaternium 39, Polyquaternium 47, and octylacrylamine/acrylates/butylaminoethyl methacrylate copolymers.

Commercially available additional water soluble polymers highly useful herein include xanthan gum with tradename Keltrol series available from Kelco, Carbomers with tradenames Carbopol 934, Carbopol 940, Carbopol 950, Carbopol 980, and Carbopol 981, all available from Noveon, acrylates/steareth-20 methacrylate copolymer with tradename ACRYSOL 22 available from Rohm and Hass, nonoxynyl hydroxyethylcellulose with tradename AMERCELL POLYMER HM-1500 available from Amerchol, methylcellulose with tradename BENECEL, hydroxyethyl cellulose with tradename NATROSOL, hydroxypropyl cellulose with tradename KLUCEL, cetyl hydroxyethyl cellulose with tradename POLYSURF 67, all supplied by Herculus, scleroglucan with tradename Clearogel SC11 available from Michel Mercier Products Inc. (NJ, USA), ethylene oxide and/or propylene oxide based polymers with tradenames CARBOWAX PEGs, POLYOX WASRs, and UCON FLUIDS, all supplied by Amerchol.

### WATER SOLUBLE HUMECTANT

The composition of the present invention may further contain from 1% to 20%, preferably from 5% to 15% of a water soluble humectant. Water soluble humectants useful herein include polyhydric alcohols such as glycerin, sorbitol, propylene glycol, butylene glycol, hexylene glycol, ethoxylated glucose, 1,2-hexane diol, 1,2-pentane diol, hexanetriol, dipropylene glycol, erythritol, trehalose, diglycerin, xylitol, maltitol, maltose, glucose, fructose, sodium chondroitin sulfate, sodium hyaluronate, sodium adenosin phosphate, sodium lactate, pyrrolidone carbonate, glucosamine, cyclodextrin, and mixtures thereof.

Water soluble humectants useful herein include water soluble alkoxylated nonionic polymers such as polyethylene glycols and polypropylene glycols having a molecular weight of up to about 1000 such as those with CTFA names PEG-200, PEG-400, PEG-600, PEG-1000, and mixtures thereof.

Commercially available humectants highly useful herein include: butylene glycol with tradename 1,3-butylene glycol available from ASAHI DENKA Co. Ltd.; glycerin with tradenames CRODEROL GA7000 available from Croda Universal Ltd., PRECERIN series available from Unichema, and a same tradename as the chemical name available from NOF; propylene glycol with tradename LEXOL PG-865/855 available from Inolex, 1,2-PROPYLENE GLYCOL USP available from BASF; dipropylene glycol with the same tradename available from BASF; diglycerin with tradename DIGLYCEROL available from Solvay GmbH; polyethylene glycols with the tradename CARBOWAX series available from Union Carbide, and a mixture of glyceryl polymethacrylate, propylene glycol and PVM/MA copolymer with tradename Lubrajel Oil available from Guardian Lab.

### SEBUM ABSORBING AGENT

The composition of the present invention may contain from 0.1% to 10%, preferably from 1% to 5% of a sebum absorbing agent. Sebum absorbing agents useful herein include those which actually absorb the sebum discreted from the pores, and are compatible with the aqueous composition of the present invention. Components which are water soluble, water swellable, or have high emulsifying ability are not suitable herein, as they would no longer have sebum absorbing ability when formulated in the composition.

Preferable sebum absorbing agents herein include porous spherical cellulose powder, solid silicone elastomer powder, surface modified porous silica powder, porous nylon powder, porous acrylate copolymer, and mixtures thereof. The type and amount of sebum absorbing agents are selected according to the desired character of the product.

Commercially available porous spherical cellulose powders highly useful herein include the materials with tradename Celluflow series, such as Celluflow C025 available from Chisso Corp. Commercially available solid silicone elastomer powders highly useful herein include vinyl dimethicone/methicone silsesquioxane crosspolymer with tradenames KSP series available from ShinEtsu Chemical Co., Ltd., Tokyo Japan. Other commercially available sebum absorbing agents include porous acrylate copolymers with tradename Polytrap available from Dow Corning.

### TACKY SKIN TREATMENT AGENT

The composition of the present invention may contain from 0.5% to 10%, preferably from 1% to 5% of a tacky skin treatment agent. Skin treatment agents useful herein are those which help repair and replenish the natural moisture barrier function of the epidermis, thereby providing skin benefits such as texture improvement. It is generally known that, while such agents provide useful benefits to the skin when used chronically, they also tend to provide negative skin feel upon use when applied by itself.

Tacky skin treatment agents useful herein are niacinamide, nicotinic acid and its esters, nicotinyl alcohol, panthenol, panthenyl ethyl ether, n-acetyl cysteine, n-acetyl-L-serine, phosphodiesterase inhibitors, trimethyl glycine, urea, gelatin, soluble collagen, royal jelly, tocopheryl nicotinate, and vitamin D3 and analogues or derivatives, and mixtures thereof. Niacinamide is particularly preferred in that, when used in a pharmaceutically effective amount, is capable of reducing or alleviating the intensity of chronical spots. Niacinamide is suitably incorporated in the composition by first dissolving in water. Panthenol is also particularly preferred in that, when used in an amount of at least about 1%, it provides texture improvement benefits. Niacinamide and panthenol are commercially available, for example, by Roche.

### SEBUM SUPRESSING PLANT EXTRACT

The composition of the present invention may further comprise from 0.001% to 5%, more preferably from 0.05% to 1% of a sebum suppressing plant extract. The plant extracts useful herein are those which have an astringent type of effect for reducing the size of pores, or inhibition effect of 5-α-reductase, and are compatible with the aqueous form of the present composition, and preferably do not alter the transparent or translucent appearance of the present composition. Water soluble plant extracts are preferred. Useful plant extracts herein include clove (choji) extract, coix (yokuinin) extract, witch hazel (hamamerisu) extract, and mixtures thereof. Such plant extracts are available from Iwase.

### WHITENING AGENT

The composition of the present invention may further comprise from 0.001% to 10%, more preferably from 0.1% to 5% of a whitening agent. Whitening agents useful herein are those which are compatible with the aqueous form of the present composition. Water soluble whitening agents are preferred. The whitening agent useful herein refers to active ingredients that not only alter the appearance of the skin, but further improve hyperpigmentation as compared to pre-treatment.

Useful whitening agents useful herein include ascorbic acid compounds, azelaic acid, butyl hydroxy anisole, gallic acid and its derivatives, glycyrrhizinic acid, hydroquinoine, kojic acid, arbutin, mulberry extract, and mixtures thereof. Use of combinations of whitening agents is believed to be advantageous in that they may provide whitening benefit through different mechanisms.

Preferably, the ascorbic acid compound useful herein is an ascorbic acid salt or derivative thereof. Exemplary water soluble salt derivatives include, but are not limited to, L-ascorbic acid 2-glucoside, L-ascorbyl phosphate ester salts such as sodium L-ascorbyl phosphate, potassium L-ascorbyl phosphate, magnesium L-ascorbyl phosphate, calcium L-ascorbyl phosphate, aluminum L-ascorbyl phosphate. L-ascorbyl sulfate ester salts can also be used. Examples are sodium L-ascorbyl sulfate, potassium L-ascorbyl sulfate, magnesium L-ascorbyl sulfate, calcium L-ascorbyl sulfate and aluminum L-ascorbyl sulfate.

### ADDITIONAL COMPONENTS

The compositions herein may further contain other additional components, which may be selected by the artisan according to the desired characteristics of the final product and which are suitable for rendering the compositions more cosmetically or aesthetically acceptable or to provide them with additional usage benefits. The components useful herein are conveniently categorized by a certain benefit or their postulated mode of action, however, a given category is not limiting of its use. Further, it is understood the one component may provide multiple benefits.

### (i) Anti-Oxidants and Radical Scavengers

Anti-oxidants and radical scavengers are especially useful for providing protection against UV radiation which can cause increased scaling or texture changes in the stratum corneum and against other environmental agents which can cause skin damage. Preferred anti-oxidants/radical scavengers include, for example, tocopherol sorbate and other esters of tocopherol, more preferably tocopherol sorbate. For example, the use of tocopherol sorbate in topical emulsions and applicable to the present invention is described in U.S. Patent 4,847,071, Bissett et al, issued July 11, 1989.

### (ii) Anti-Inflammatory Agents

Anti-inflammatory agents enhance the skin appearance benefits, by for example, contribution of uniformity and acceptable skin tone and/or color. Preferably, the anti-inflammatory agent includes a steroidal anti-inflammatory agent and a non-steroidal anti-inflammatory agent. Preferred steroidal anti-inflammatory for use is hydrocortisone. So-called "natural" anti-inflammatory agents are also useful. For example, alpha bisabolol, aloe vera, Manjistha (extracted from plants in the genus Rubia, particularly Rubia Cordifolia), and Guggal (extracted from plants in the genus Commiphora, particularly Commiphora Mukul), kola extract, chamomile, and sea whip extract, may be used.

### (iii) Antimicrobial Agent

As used, "antimicrobial agents" means a compound capable of destroying microbes, preventing the development of microbes or preventing the pathogenic action of microbes. Antimicrobal agents are useful, for example, in controlling acne. Preferred antimicrobial agents useful in the present invention are benzoyl peroxide, erythromycin, tetracycline, clindamycin, azelaic acid, sulfur resorcinol, phenoxyethanol, and Irgasan™ DP 300 (Ciba Geigy Corp., U.S.A.). A safe and effective amount of an antimicrobial agent may be added to emulsions of the present invention, preferably from 0.001% to 10%, more preferably from 0.01% to 5%, still more preferably from 0.05% to 2%.

### (iv) Chelators

As used herein, "chelator" refers to a compound that reacts for removing a metal ion from a system by forming a complex so that the metal ion cannot readily participate in or catalyze chemical reactions. The inclusion of a chelator is especially useful for providing protection against UV radiation which can contribute to excessive scaling or skin texture changes and against other environmental agents which can cause skin damage. Exemplary chelators that are useful herein are disclosed in U.S. Patent 5,487,884, Bissett et al, issued January 30, 1996; PCT application 91/16035 and 91/16034, Bush et al, published October 31, 1995. Preferred chelators are furildioxime and derivatives thereof.

### (v) Silicone Elastomers

The compositions of the present invention may include a non-emulsifying crosslinked organopolysiloxane elastomer. The term "non-emulsifying," as used herein, defines crosslinked organopolysiloxane elastomers from which polyoxyalkylene units are absent. Such elastomers are used to reduce the tackiness/stickiness feel associated with skin conditioning agents.

Advantageously, the elastomers may be dimethicone/vinyl dimethicone crosspolymers, vinyl dimethicone/lauryl dimethicone crosspolymers, C30-C45, alkyl ceteayl dimethicone/polycyclohexane oxide crosspolymers, and mixtures thereof.

Dimethicone/vinyl dimethicone crosspolymers are supplied by a variety of suppliers including Dow Coming (DC 9040 and DC 9041), General Electric (SFE 839), Shin Etsu (KSG-15, 16, 18 [dimethicone/phenyl vinyl dimethicone Crosspolymer]), and Grant Industries (GRANSIL™ line of elastomers). Cross-linked organopolysiloxane elastomers useful in the present invention and processes for making them are further described in U.S. Patent 4,970,252 to Sakuta, et al., issued November 13, 1990; U.S. Patent 5,760,116 to Kilgour, et al., issued June 2, 1998; U.S. Patent 5,654,362 to Schulz, Jr., et al. issued August 5, 1997 .

The vinyl dimethicone/lauryl dimethicone crosspolymers include vinyl dimethicone/lauryl dimethicone crosspolymer & mineral oil (tradename KSG-41); vinyl dimethicone/lauryl dimethicone crosspolymer & isododecane (tradename KSG-42); vinyl dimethicone/lauryl dimethicone crosspolymer & triethylhexanoin (tradename KSG-43); vinyl dimethicone/lauryl dimethicone crosspolymer & squalane (tradename KSG-44). Each of these "KSG" denominated silicone elastomers is available from Shinestu Chemical.

Commercially available cyclomethicone and C30-C45 alkyl ceteayl dimethicone/polycyclohexane oxide crosspolymer is available from GE Silicone under the tradename Velvasil 125.

### (vi) Other Components

In addition to the above described components, the composition of the present invention may further include preservatives and preservative enhancers such as water-soluble or solubilizable preservatives including Germall 115, methyl, ethyl, propyl and butyl esters of hydroxybenzoic acid, benzyl alcohol, imidazolidinyl urea, EDTA and its salts, Bronopol (2-bromo-2-nitropropane-1,3-diol) and phenoxypropanol; antifoaming agents; binders; biological additives; bulking agents; coloring agents; essential oils and solubilizers thereof; other natural extracts; compounds which stimulate collagen production; yeast fermented filtrates, and others.

### EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention. Where applicable, ingredients are identified by chemical or CTFA name, or otherwise defined below.

### Compositions

| Phase | | Ex.1 | Ex.2 | Ex.3 | Ex.4 | Ex.5 | Ex.6 | Ex.7 |
|---|---|---|---|---|---|---|---|---|
| 01 | Butyl Methoxy Dibenzoylmethane | 2 | 2 | 1.5 | 1.5 | 2 | 2 | 3 |
| 01 | Butyloctyl Salicylate | - | 4 | 2 | - | - | - | - |
| 01 | Octocrylene | 3 | 3 | 2 | 1.5 | 3 | 3 | 2.5 |
| 01 | Octyl Salicylate | 5 | 5 | 4 | 4 | 3 | 3 | - |
| 01 | Homosalate | - | - | - | - | - | - | 7 |
| 01 | Isopropyl lauroyl sarcosinate | 5 | - | - | 4 | 3 | 3 | 6 |
| 01 | Diethyl hexyl 2,6-naphthalate | - | - | - | - | - | 4 | - |
| 01 | Tricaprylin | - | - | - | - | 3 | - | - |
| 02 | Dimethicone PEG-7 isostearate *1 | - | 0.2 | 0.2 | 0.1 | - | - | - |
| 02 | PEG-10 Dimethicone *2 | - | - | - | - | 0.1 | 0.1 | - |
| 02 | Dimethicone PEG-7 olivate *3 | - | - | - | - | - | 0.1 | - |
| 02 | Methyl methacrylate crosspolymer *4 | 2 | 1 | 1 | - | - | 1 | 0.5 |
| 02 | Phenyl Trimethicone *5 | 1 | 1 | 1 | - | - | - | - |
| 02 | Phenyl propyl trimethicone *6 | - | - | - | 1 | 1 | 1 | - |
| 02 | Cyclomethicone and C30-C45 Alkyl Ceteayl Dimethicone/Polycyclohexane Oxide Crosspolymer*7 | - | - | 3 | - | 5 | - | 5 |
| 02 | Vinyl Dimethicone/Lauryl Dimethicone Crosspolymer & Isododecane*8 | - | - | - | - | - | 5 | 5 |
| 02 | Vinyl Dimethicone/Lauryl Dimethicone Crosspolymer & Triethylhexanoin*9 | - | 7 | - | - | - | - | - |
| 03 | Sodium Polyacrylate*10 | - | - | 0.6 | - | - | - | 0.55 |
| 03 | Ammonium Acryloyldimethyl taurate/VP Copolymer *11 | - | - | - | 0.65 | - | - | - |
| 03 | Acrylic acid/alkyl acrylate copolymer 1 *12 | 0.2 | - | - | 0.2 | 0.16 | - | - |
| 03 | Acrylic acid/alkyl acrylate copolymer 2 *13 | - | 0.16 | 0.16 | - | - | 0.2 | |
| 03 | De ionized Water | balance to 100% | | | | | | |
| 04 | Amino Methylpropanol | 0.15 | 0.1 | 0.1 | 0.15 | 0.1 | 0.15 | - |
| 05 | Polysorbate-20 | 0.12 | 0.2 | 0.2 | 0.15 | - | - | 0.2 |
| 05 | Polysorbate-40 | 0.08 | - | - | 0.05 | - | - | - |
| 05 | PEG-60 hydrogenated castor oil | - | - | - | - | - | 0.2 | - |
| 05 | PEG-20 Isohexadecyl ether | - | - | - | - | 0.2 | - | - |
| 06 | 1,3-Butylene glycol | 1 | 1 | 1 | 1 | 1 | 1 | 1 |
| 06 | Glycerin | - | 2 | - | 2 | - | - | 5 |
| 06 | Methyl Paraben | - | 0.1 | 0.1 | - | 0.1 | - | 0.1 |
| 07 | Deionized water | 10 | 10 | 10 | 10 | 10 | 10 | 10 |
| 07 | Niacinamide | 3 | 2 | 2 | 3 | 2 | 2 | 4 |
| 08 | Menthol | - | 0.1 | 0.1 | - | 0.1 | - | - |
| 08 | Perfume | 0.05 | 0.075 | 0.1 | 0.1 | 0.075 | 0.05 | 0.125 |
| 08 | Ethanol | 10 | 10 | 10 | 10 | 5 | 8 | 10 |
| 09 | Sodium Hyaluronate | 0.05 | - | - | - | 0.02 | 0.02 | - |
| 09 | D-panthenol | - | 1 | 1 | 1 | 2 | 1 | 0.5 |
| 09 | Zizyphus Jujuba Fruit Extract *14 | 0.1 | - | 0.15 | 0.1 | - | 0.15 | - |
| 09 | Witch Hazel Extract *15 | - | - | - | 0.1 | - | - | - |
| 09 | Clove Extract *16 | - | - | 0.1 | - | - | - | - |
| 09 | Magnesium Ascorbyl Glucoside | - | - | - | - | 2 | - | - |
| 09 | Titanium Dioxide *17 | - | - | - | - | - | 1 | - |
| 09 | Cellulose Powder *18 | - | - | - | - | 3 | - | - |
| 09 | Xanthan gum | - | - | - | - | - | 0.5 | - |
| 09 | Di-sodium EDTA | 0.1 | - | - | 0.1 | 0.05 | 0.05 | - |
| 09 | De ionized water | 10 | 10 | 10 | 10 | 10 | 10 | 10 |

| | | | | | | | | |
|---|---|---|---|---|---|---|---|---|
| Definitions of Components *1 Dimethicone PEG-7 Isostearate: Ultrasil DW18 available: from Noveon. *2 PEG-10 Dimethicone: KF-6017 available from Shinetsu Chem. *3 Dimethicone PEG-7 olivate: Ultrasil DW-O available from Noveon *4 Methyl methacrylate crosspolymer: GanzPearl 2001 available from Ganz Kasei *5 Phenyl Trimethicone: Silicone Oil KF56 available from Shinetsu Chem. *6 Phenyl propyl trimethicone: DC2-2043 Cosmetic Grade Fluid available from Dow Corning *7 Cyclomethicone and C30-C45 Alkyl Ceteayl Dimethicone/Polycyclohexane Oxide Crosspolymer: Velvasil 125 available from GE Silicone *8 Vinyl Dimethicone/Lauryl Dimethicone Crosspolymer & Isododecane: KSG-42 available from Shinetsu Chem. *9 Vinyl Dimethicone/Lauryl Dimethicone Crosspolymer & Triethylhexanoin: KSG-43 available from Shinetsu Chem. *10 SodiumPolyacrylate: Cosmedia SP available from Cognis *11 Ammonium Acryloyldimethyl taurate/VP Copolymer: Aristoflex AVC available from Clariant *12 Acrylic acid/alkyl acrylate copolymer 1: Carbopol ETD 2020 available from Noveon. *13 Acrylic acid/alkyl acrylate copolymer 2: Carbopol Ultrez 21 available from Noveon. *14 Zizyphus Jujuba Fruit Extract: Available from Ichimaru Pharcos *15 Witch Hazel Extract: Hamamerisu Liquid available from Iwase *16 Clove Extract: Choji Extract BG available from Iwase *17 Titanium Dioxide: Titanium Dioxide MT-100 available from Tayca *18 Cellulose Powder: Celluflow C-25 available from Chisso corp. | | | | | | | | |

### Method of Preparation

The skin care compositions of "Ex. 1" through "Ex. 7" as shown above can be prepared any conventional method well known in the art. For example, the compositions of the present invention can be prepared by following steps:
(a) Hear Phase 03 to about 80°C and mix until polymers are dissolved. Then cool the Phase 03 to about 25°C.
(b) Add Phase 04 to mixture of (a).
(c) Add Phase 05 and Phase 06 to the mixture of (b).
(d) Add Phase 01 and Phase 02 to the mixture of (c). When a solid UV protecting agent is contained in Phase 01, it is dissolved in a solvent at about 65°C before Phase 01 is added to the mixture of (c).
(e) Add Phase 07 to the mixture of (d).
(f) Finally, Phase 08 and Phase 09 to the mixture of (e).

The embodiments disclosed and represented by the previous "Ex. 1" through "Ex. 7" have many advantages. For example, Examples 1 through 7 are particularly useful for providing UV protecting benefit to the skin. When used on the facial skin, the compositions of Examples 1 through 7 can provide UV protecting benefit without leaving a tacky and/or greasy feel to the skin.

## Claims

1. A skin care composition comprising:
(a) from 0.001% to 5.0% of a hydrophilic gelling agent;
(b) from 0.001% to 1.0% of a first nonionic surfactant system having a HLB value of 10 or more, wherein the first surfactant system comprises one or more nonionic surfactants selected from the group consisting of polysorbates, polyoxyalkylene hydrogenated caster oils, polyglycerin alkyl esters having the C10-20 of alkylsubstitute, polyoxyethylene sterols, and polyoxyethylene hydrogenated sterols;
(c) from 3% to 30% of a lower alcohol;
(d) from 5% to 40% of an oily component; and
(e) water
wherein, the weight ratio of the oily component to the first surfactant system is in the range from 10:1 to 100:1 but when second and further surfactants are contained in the composition the weight ratio of the oily component to total surfactants is in the range from 10:1 to 100:1; and
wherein the composition comprises, as (c), from 3% to 30% of a lower alcohol selected from ethanol and isopropanol.

2. The skin care composition of Claim 1, wherein the hydrophilic gelling agent is selected from the group consisting of carboxylic acid/carboxylate copolymers, acrylate homopolymers, acrylate copolymers, and mixtures thereof.

3. The skin care composition of Claim 1, wherein the first surfactant system is liquid at 40°C.

4. The skin care composition of Claim 1, wherein the first surfactant system has an HLB of 12 or more.

5. The skin care composition of Claim 1, wherein the first surfactant system comprises a polysorbate selected from the group consisting of polysorbate-20, polysorbate-40, and mixtures thereof.

6. The skin care composition of Claim 1, wherein the oily component is selected from the group consisting of UV protecting agents, solvents of the UV protecting agents, emollients, silicone oils, and mixtures thereof.

7. The skin care composition of Claim 6, wherein the first oily component comprises UV agents and solvents of the UV agents, wherein the UV agents is selected from the group consisting of octyl methoxylcinnamate, octyl salicylate, octocrylene, avobenzone, homosalate, octyl triazone, and mixtures thereof.

8. The skin care composition of claim 1 comprising from 10 to 25% by weight of said oily component.

9. The skin care composition of Claim 1, further comprising from 0.001% to 1.0% of a second nonionic surfactant system selected from the group consisting of silicone copolyol ester, silicone copolyol, and mixtures thereof.

10. The skin care composition of Claim 9, wherein the second surfactant system comprises a silicone copolyol ester.

11. The skin care composition of Claim 9, wherein the second surfactant system is liquid at 40°C.

12. The skin care composition of Claim 1 further comprising from 0.01% to 5% of an additional water soluble polymer.

13. The skin care composition of Claim 1 further comprising from 1% to 20% of an additional water soluble humectant.

## Patentansprüche

1. Hautpflegezusammensetzung, umfassend:
(a) von 0,001 % bis 5,0 % ein hydrophiles Geliermittel;
(b) von 0,001 % bis 1,0 % ein erstes nichtionisches Tensidsystem mit einem HLB-Wert von 10 oder mehr, wobei das erste Tensidsystem ein oder mehrere nichtionische Tenside umfasst, die ausgewählt sind aus der Gruppe bestehend aus Polysorbaten, hydrierten Polyoxyalkylenrizinusölen, Polyglycerinalkylestern mit C10-20-Alkylsubstitution, Polyoxyethylensterolen und hydrierten Polyoxyethylensterolen;
(c) von 3 % bis 30 % einen niederen Alkohol;
(d) von 5 % bis 40 % einen öligen Bestandteil; und
(e) Wasser
wobei das Gewichtsverhältnis des öligen Bestandteils zu dem ersten Tensidsystem im Bereich von 10:1 bis 100:1 liegt, wobei jedoch, wenn das zweite und weitere Tenside in der Zusammensetzung enthalten sind, das Gewichtsverhältnis des öligen Bestandteils zu gesamten Tensiden im Bereich von 10:1 bis 100:1 liegt; und
wobei die Zusammensetzung als (c) von 3 % bis 30 % einen niederen Alkohol, ausgewählt aus Ethanol und Isopropanol, umfasst.

2. Hautpflegezusammensetzung nach Anspruch 1, wobei das hydrophile Geliermittel ausgewählt ist aus der Gruppe bestehend aus Carbonsäure/Carboxylat-Copolymeren, Acrylathomopolymeren, Acrylatcopolymeren und Mischungen davon.

3. Hautpflegezusammensetzung nach Anspruch 1, wobei das erste Tensidsystem bei 40 °C flüssig ist.

4. Hautpflegezusammensetzung nach Anspruch 1, wobei das erste Tensidsystem einen HLB von 12 oder mehr aufweist.

5. Hautpflegezusammensetzung nach Anspruch 1, wobei das erste Tensidsystem ein Polysorbat umfasst, das ausgewählt ist aus der Gruppe bestehend aus Polysorbat-20, Polysorbat-40 und Mischungen davon.

6. Hautpflegezusammensetzung nach Anspruch 1, wobei der ölige Bestandteil ausgewählt ist aus der Gruppe bestehend aus UV-Schutzmitteln, Lösungsmitteln der UV-Schutzmittel, Weichmachern, Silikonölen und Mischungen davon.

7. Hautpflegezusammensetzung nach Anspruch 6, wobei der erste ölige Bestandteil UV-Mittel und Lösungsmittel der UV-Mittel umfasst, wobei die UV-Mittel ausgewählt sind aus der Gruppe bestehend aus Octylmethoxylcinnamat, Octylsalicylat, Octocrylen, Avobenzon, Homosalat, Octyltriazon und Mischungen davon.

8. Hautpflegezusammensetzung nach Anspruch 1, umfassend von 10 bis 25 Gew.-% den öligen Bestandteil.

9. Hautpflegezusammensetzung nach Anspruch 1, ferner umfassend von 0,001 % bis 1,0 % ein zweites nichtionisches Tensidsystem, ausgewählt aus der Gruppe bestehend aus Silikon-Copolyolester, Silikon-Copolyol und Mischungen davon.

10. Hautpflegezusammensetzung nach Anspruch 9, wobei das zweite Tensidsystem einen Silikon-Copolyolester umfasst.

11. Hautpflegezusammensetzung nach Anspruch 9, wobei das zweite Tensidsystem bei 40 °C flüssig ist.

12. Hautpflegezusammensetzung nach Anspruch 1, ferner umfassend von 0,01 % bis 5 % ein zusätzliches wasserlösliches Polymer.

13. Hautpflegezusammensetzung nach Anspruch 1, ferner umfassend von 1 % bis 20 % ein zusätzliches wasserlösliches Feuchthaltemittel.

## Revendications

1. Composition de soin de la peau comprenant :
(a) de 0,001 % à 5,0 % d'un agent gélifiant hydrophile ;
(b) de 0,001 % à 1,0 % d'un premier système tensioactif non ionique ayant un rapport hydro-lipophile de 10 ou plus, dans laquelle le premier système tensioactif comprend un ou plusieurs agents tensioactifs non ioniques choisis dans le groupe constitué de polysorbates, huiles de ricin hydrogénées polyoxyalkylène, esters d'alkyle de polyglycérine ayant la substitution alkyle en C10 à 20, des polyoxyéthylène stérols, et des polyoxyéthylène stérols hydrogénés ;
(c) de 3 % à 30 % d'un alcool inférieur ;
(d) de 5 % à 40 % d'un composant huileux ; et
(e) de l'eau
dans laquelle, le rapport pondéral du composant huileux sur le premier système tensioactif est de l'ordre de 10:1 à 100:1, mais lorsque des deuxièmes et autres agents tensioactifs sont contenus dans la composition, le rapport pondéral du composant huileux sur les agents tensioactifs totaux est de l'ordre de 10:1 à 100:1. et
dans laquelle la composition comprend, pour (c), de 3 % à 30 % d'un alcool inférieur choisi entre l'éthanol et l'isopropanol.

2. Composition de soin de la peau selon la revendication 1, dans laquelle l'agent gélifiant hydrophile est choisi dans le groupe constitué de copolymères acide carboxylique/carboxylate, homopolymères acrylate, copolymères acrylate, et leurs mélanges.

3. Composition de soin de la peau selon la revendication 1, dans laquelle le premier système tensioactif est liquide à 40 °C.

4. Composition de soin de la peau selon la revendication 1, dans laquelle le premier système tensioactif a un rapport hydrophile-lipophile de 12 ou plus.

5. Composition de soin de la peau selon la revendication 1, dans laquelle le premier système tensioactif comprend un polysorbate choisi dans le groupe constitué de polysorbate-20, polysorbate-40, et leurs mélanges.

6. Composition de soin de la peau selon la revendication 1, dans laquelle le composant huileux est choisi dans le groupe constitué d'agents de protection contre les UV, solvants des agents de protection contre les UV, émollients, huiles de silicone, et leurs mélanges.

7. Composition de soin de la peau selon la revendication 6, dans laquelle le premier composant huileux comprend des agents contre les UV et des solvants des agents contre les UV, dans laquelle les agents contre les UV sont choisis dans le groupe constitué de méthoxylcinnamate d'octyle, salicylate d'octyle, octocrylène, avobenzone, homosalate, octyl triazone, et leurs mélanges.

8. Composition de soin de la peau selon la revendication 1, comprenant de 10 % à 25 % en poids dudit composant huileux.

9. Composition de soin de la peau selon la revendication 1, comprenant en outre de 0,001 % à 1,0 % d'un deuxième système tensioactif non ionique choisi dans le groupe constitué d'ester de copolyol de silicone, copolyol de silicone, et des mélanges de ceux-ci.

10. Composition de soin de la peau selon la revendication 9, dans laquelle le deuxième système tensioactif comprend un ester de copolyol de silicone.

11. Composition de soin de la peau selon la revendication 9, dans laquelle le deuxième système tensioactif est liquide à 40 °C.

12. Composition de soin de la peau selon la revendication 1, comprenant en outre de 0,01 % à 5 % d'un polymère hydrosoluble supplémentaire.

13. Composition de soin de la peau selon la revendication 1, comprenant en outre de 1 % à 20 % d'un humectant hydrosoluble supplémentaire.
